Europäisches Patentamt

European Patent Office

Office européen des brevets

(11) Publication number : **0 468 702 A2**

## (12) EUROPEAN PATENT APPLICATION

(21) Application number : **91306541.3**

(22) Date of filing : **18.07.91**

(51) Int. Cl.$^5$ : **C12N 7/02, A61K 39/29**

(30) Priority : **18.07.90 US 555020**

(43) Date of publication of application :
**29.01.92 Bulletin 92/05**

(84) Designated Contracting States :
**CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Lewis, John A.**
**1229 Clearbrook Road**
**West Chester, PA 19380 (US)**
Inventor : **Armstrong, Marcy Ellen**
**1080 Barnbridge Drive**
**Schwenksville, PA 19473 (US)**

(74) Representative : **Barrett-Major, Julie Diane et al**
**Merck & Co., Inc. European Patent Department Terlings Park Eastwick Road Harlow Essex CM20 2QR (GB)**

(54) **Novel process for purification of Hepatitis-A virus capsids.**

(57)   New methods for purifying Hepatitis-A virus (HAV) capsids are amenable to commercial scale-up and manufacture of specific HAV vaccines, including formalin-inactivated HAV and attenuated HAV.

EP 0 468 702 A2

## BACKGROUND OF THE INVENTION

Hepatitis A virus (HAV) is a morphologically, biochemically, and immunologically distinct picornavirus that is the etiological agent of infectious hepatitis in humans.

Various methods have been described to partially purify HAV virions for investigation and initial virion characterization. See, for example, Hornbeck, C.L. et al., Intervirology 6; 309-314 (1975); Locarnini, S.A. et al., Intervirology 10; 300-308 (1978); Siegl, G. et al., J. Virol. 26, 40-47 (1978); Siegl, G. et al., J. Gen. Virol. 57, 331-341 (1981); Siegl, G. et al., Intervirology 22, 218 (1984); Hughes, J.V. et al., J. Virol. 52, 465 (1984); and Wheeler, C.M. et al., J. Virol, 58, 307 (1986). Each of these methods employs one or more steps that may hinder approval by the Food and Drug Administration, of any HAV vaccine for human use, or are commerically impractical. For example, detergents and/or exogenous enzymes are commonly used, and, all of these methods employ unwieldy, impractical and excessively expensive steps, such as sucrose gradient centrifugation or CsCl-density gradient centrifugation.

Applicants have discovered methods of obtaining extremely pure HAV capsids without the use of detergents or exogenous enzymes. Furthermore, the methods disclosed herein can be readily scaled to commercial production levels. In addition, applicants have developed these methods for MRC-5 human diploid fibroblast cell culture , which are certified by the Food and Drug Administration for use in human vaccine production.

## BRIEF DESCRIPTION OF THE INVENTION

Methods of substantially purifying HAV capsids are disclosed, comprising the steps of:
(a) subjecting the aqueous phase of a lysate of HAV infected cells to ion exchange chromatography;
(b) subjecting the effluent of (a) to ion exchange chromatography; and
(c) gel filtering the eluate of (b) containing HAV capsids, yielding substantially purified HAV capsids.

## BRIEF DESCRIPTION OF THE DRAWINGS

Figure 1 - electron micrograph (magnification 130,000 x) of the effluent from the first anion exchange chromatography step comprising HAV capsids (c) and 30 nm particles (p).

Figure 2 - electron micrograph (magnification 130,000 x) of the eluate from the second anion exchange chromatographic step comprising HAV capsids (c) substantially free of the 30 nm particles.

## DETAILED DESCRIPTION OF THE INVENTION

The commercially adaptable purification processes of the present invention encompass the purification of any HAV capsids, whether derived from attenuated or virulent HAV, produced in any cell line or culture susceptible to infection by HAV. In addition, all HAV capsids, whether empty (without viral nucleic acids) or complete (containing viral nucleic acids) are encompassed within the process of the present invention.

HAV variant P18 of strain CR326F was used to infect MRC-5 cells in this invention, for merely illustrative purposes. P18CR326F is an attenuated HAV strain. Other strains and/or serotypes of HAV capsids are encompassed by this invention, including HAV strains that can be attenuated by conventional techniques. Other suitable cell lines for HAV propagation include Vero, FL, WI-38 and FRhK6 cells. These and other systems for HAV propagation in cell cultures are discussed in Gerety, R.J. "Active Immunization Against Hepatitis A," in Gerety, R.J. (ed.) Hepatitis A Academic Press 1984, pp. 263-276; and Ticehurst, J.R., seminars in Liver Disease 6,46-55 (1986). In principle, any cell line such as any human diploid fibroblast cell line, can serve as a host cell for HAV provided that it is susceptible to HAV infection. The preferred cell line is MRC-5.

HAV-infected cells are grown and harvested by any one or more of a variety of conventional techniques. Growth conditions, including the selection of media, depend on the requirements of the cell line employed. MRC-5 cells are anchorage dependent, therefore, attachment to a substratum is necessary, which requires detachment of the cells by physical, chemical or enzymatic means for harvesting. Other cell types useful for the purposes of this invention include those capable of anchorage independent growth, which are usually harvested by centrifugation.

After growth and harvest, the HAV-infected cells are lysed by any one or more of a variety of techniques, including but not limited to exposure to hypotonic buffer, vortexing, freeze-thaw cycling, and sonication. The resulting suspension of disrupted cells, whether the cells were disrupted by any one or combination of cell lysis techniques, is hereinafter referred to as a "cell lysate".

Applicants have found that sonication is the preferred lysis technique for sufficiently complete lysis of HAV-infected MRC-5 cells resulting in efficient release of HAV capsids from the cell. (Any release of HAV capsids from a disrupted host cell is hereinafter referred to as "lysis".) It is thought that sonication efficiently releases HAV capsids from the subcellular pseudocrystalline state within the cell. Even more preferred is a combination of sonication and one or more other lysis techniques such as hypotonic lysis, vortexing or freeze-thaw cycling. Applicants most prefer the following steps in lysing HAV infected MRC-5 cells:

(1) thawing harvested frozen cells in tubes, and adding lysis buffer (10 mM TRIS-HCl pH 7.5, 10 mM NaCl, 1.5 mM $MgCl_2$);

(2) vigorously vortexing (10-15 seconds at setting 10 using a Vortex-Genie);

(3) freeze-thaw cycling one or more times by successive freezing in an ethanol-dry ice bath, followed by thawing in a 37°C water bath; and

(4) sonicating one or more times for 30 seconds at the maximum wattage output of a cup horn sonicator with a circulating ice water bath (Branson sonifier Cell Disrupter 185 and Ultrasonics cup horn).

The cell lysate is diluted with buffer and then subjected to organic extraction. Such extraction removes, among other contaminants, lipid and lipid-like substances. Accordingly, the cell lysate is diluted with any one of a variety of buffers, including phosphate buffered saline, TRIS, carbonate, bicarbonate, or acetate buffers. Since HAV capsids are probably acid stable, buffers in an acid pH range are also feasible substitutes. Applicants have discovered that the presence of $MgCl_2$ in the buffer substantially lowers HAV capsid yields. The most preferable buffer is TNE (10 mM TRIS-HCl pH 7.5, 150 mM NaCl, 1 mM EDTA).

Cell lysates diluted with buffer are then extracted by the addition of a mixture of a halogenated lower alkane, such as methylene chloride, chloroform, tetrachloroethane, or the like, and an antifoaming agent, such as isoamyl alcohol. In this invention, lower alkane contains 1-6 carbon atoms. The volume-to-volume ratio of halogenated lower alkane to antifoaming agent is between approximately 15:1 and 50:1, preferably between approximately 20:1 and 30:1. Applicants have found that methylene chloride is superior to chloroform at this stage. HAV capsids are predominantly found in the aqueous phase and the aqueous/organic interface.

Most preferably, the sonicate is organically extracted by the addition of an equal volume of methylene chloride: isoamyl alcohol (24:1,v/v) with vortexing for 1 minute, and is centrifuged at 3,000 rpm for 10 minutes at 20°C in an IEC centrifuge (3,000 x g) to achieve aqueous/organic phase separation. The aqueous phase is reserved, the organic phase discarded and the interface is reextracted with a volume of TNE buffer equal to one third of the original sample volume, followed by vortexing and centrifugation as above. The two aqueous phases are pooled and the volume measured, yielding HAV capsids in organically extracted cell lysates.

The next step involves concentration with a water-soluble synthetic polymer effective to precipitate proteins. Applicants prefer to concentrate the organically extracted cell lysates (which contain HAV capsids) with polyethylene glycol (PEG) having a molecular weight of between approximately 2,000 daltons and 12,000 daltons. Typically, NaCl is added to the cell lysate to a final concentration of between approximately 150 mM and 500 mM. PEG is then added to a final concentration of between approximately 2% (w/v) and 10% (w/v). It is most preferable to adjust the organically extracted cell lysate to about 500 mM NaCl, then add PEG (8000 mw) to about 4% (w/v). The resulting 4% PEG precipitated cell lysate is centrifuged, the supernatant is discarded and the pellet resuspended for further processing. The resuspended PEG pellet is preferably sonicated before the second organic extraction, described below.

The resuspended, sonicated PEG pellet from above is extracted by the addition of a mixture of a halogenated lower alkane, such as chloroform, and an antifoaming agent, such as isoamyl alcohol. The volume-to-volume ratio of halogenated lower alkane to antifoaming agent is between approximately 15:1 and 50:1, preferably between about 20:1 and 30:1. Applicants have discovered that this second organic extraction substantially enhances purity of the final HAV product. Futhermore, in contrast to the first organicextraction applicants have found that chloroform is superior to methylene chloride in the second organic extraction.

Most preferably, the steps of the second organic extraction are carried out as follows:

The resuspended sonicated PEG pellet is then organically extracted by the addition of an equal volume of chloroform/isoamyl alcohol (24:1,v/v) with vigorous vortexing. This extract is then clarified by centrifugation at 4,000 g for 10 minutes at 20°C. The aqueous phase is reserved, the interface and organic phase are reextracted with a volume of TNE buffer equal to one third of the original sample volume, and both aqueous phases are combined, yielding extracted PEG pellet.

The extract from above is subjected to anion exchange chromatography on a resin, gel or matrix. Typical anion exchange matrices include, but are not limited to,

DEAE cellulose;

DEAE agarose;

DEAE Biogel;

DEAE dextran;

DEAE Sephadex;

DEAE Sepharose;

Aminohexyl Sepharose;

Ecteola cellulose;

TEAE cellulose;

QAE cellulose;

Mono-Q; or

Benzoylated diethylaminoethyl cellulose.

The preferred anion exchange matrix is DEAE Sepharose CL-6B (Pharmacia). General background information on ion exchange chromatography can be found, for example, in E. A. Peterson, "Cellulosic Ion Exchangers" in Work, T. S. et al. Laboratory Techniques in Biochemistry and Molecular Biology Volume 2, Part II, pages 223 et seq. North-Holland 1970.

NaCl is added to the extract to a final concentration between approximately 0.3 M to 0.4 M, with 0.35 M NaCl preferred. The extract is then passed through DEAE Sepharose CL-6B by column chromatography. The HAV capsids pass through the DEAE sepharose CL-6B without becoming adsorbed to it, while contaminants including cellular nucleic acids, are adsorbed onto the DEAE Sepharose CL-6B and remain in the column. The effluent (containing the HAV capsids) is collected, and consists of HAV capsids substantially free from cellular nucleic acids.

The effect of this anion exchange step is the removal of cellular DNA, RNA, and other impurities, from the HAV capsids. In principle, therefore, anion exchange can be substituted by the addition and removal of DNAse, or by alternative treatments designed to remove DNA or other nucleic acids at this stage of the purification process.

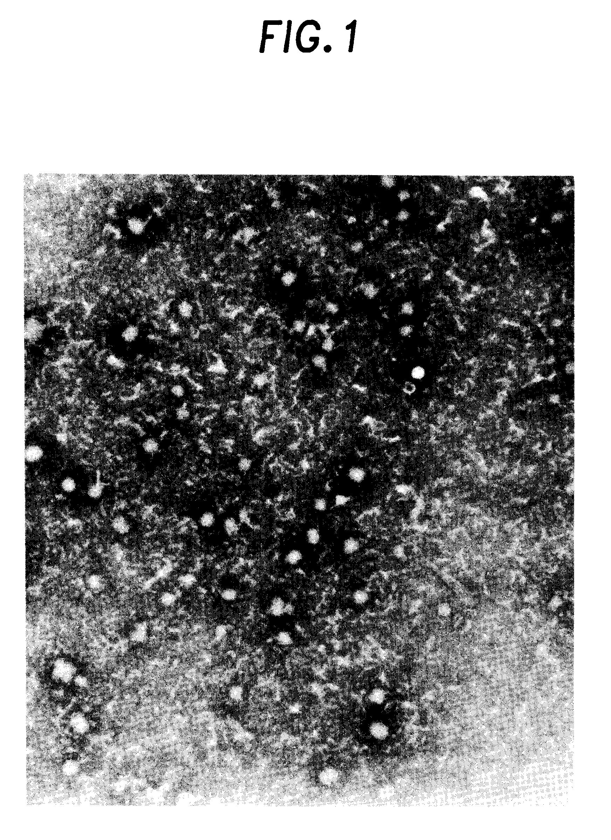

At this stage the effluent appears to be comprised of HAV capsids (both empty and complete) and diffuse particles of about 30 nm when observed by electron microscopy (see Figure 1).

The effluent from the above anion exchange chromatography which contains HAV capsids is subjected to a second anion exchange chromatographic step to further remove contaminating cellular components. The effluent is diluted with 10 mM TRIS, 1 mM EDTA, pH 7.5, to yield a NaCl concentration between approximately 0.1 M and 0.2 M, with 0.15 M NaCl preferred. The diluted effluent is passed through an anion exchange matrix. Typical anion echange matrices include, but are not limited to:

DEAE cellulose;
DEAE agarose;
DEAE biogel;
DEAE deritran;
DEAE Sephadex;
DEAE Sepharose;
Aminohexyl Sepharose;
Ecteola cellulose;
TEAE cellulose;
QAE cellulose;
Mono Q; or
Benzoylated diethylaminoethyl cellulose. The preferred anion exchange matrix is DEAE Sepharose CL-6B (Pharmacea).

It has been discovered that at the NaCl molarity described above, HAV capsids adhere to the anion exchange matrix while certain remaining contaminants pass through the matrix. The column is washed with several column volumes of 0.15 M NaCl to further remove remaining contaminants.

The HAV capsids are eluted from the anion exchange column using one column volume plus the original sample volume of 0.35 M NaCl.

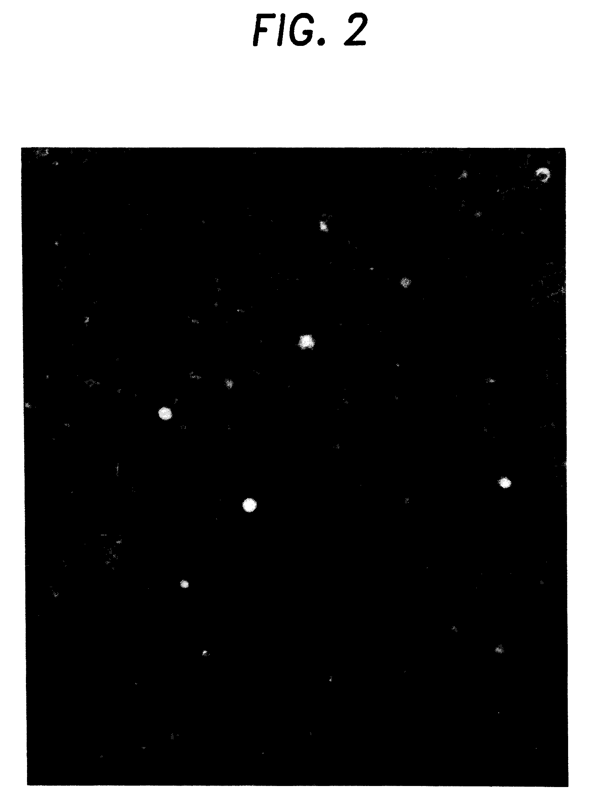

The effect of this second anion exchange step is the removal of particles which appear by electron microscopy to be diffuse, and about 30nm in diameter, that are believed to be composed of cellular carbohydrate (see Figure 2).

A final step of gel filtration chromatography follows anion exchange chromatography. Typically, Sepharose CL-4B (Pharmacia) is employed, but numerous other types of gel filtration matrices can be substituted. See, for example, Fischer, L., "Gel Filtration . Chromatography," in Work, T. S. et al. Laboratory Techniques in Biochemistry and Molecular Biology Elsevier (1980).

While the particular sequence of chromatographic steps of anion exchange followed by gel filtration are the typical protocols for purifying HAV capsids in this invention, it will be understood that the sequence can be varied. For example, gel filtration may precede either of the anion exchange steps. Furthermore, the sequence of the two anion exchange chromatography steps can be varied.

## ADDITIONAL STEPS

Other conventional or known procedures normally used in purification of proteins whether viral or cellular, or paticles such as capsids, may be optionally added to the present process of purifying HAV capsids without significantly effecting the outcome of this process. These procedures include, but are not limited to:

(a) selective adsorption or partition on a solid-phase, e.g. silica gel, calcium phosphate charcoal, or celite alumina;
(b) hydrophobic chromatography with, e.g. butyl agarose;
(c) selective extraction with other organic solvents or reagents.
(d) additional HAV capsid precipitation steps;
(e) chromatography by any standard method, including thin-layer, gel, molecular sieve, molecular exclusion, ion-exchange, ligand affinity, immuno-affinity, or by electrophoresis;
(f) solvent fractionation by two phase extractions, e.g. with PEG and dextran;
(g) dialysis, ultrafiltration, or diafiltration;
(h) density-gradient centrifugation;
(i) electrofocusing;
(j) freeze drying, or lyophilization;
(k) crystallization;
(l) addition of protease inhibitors and/or chelating agents to buffer solutions; or
(m) substitution of one buffer for another.

The above list is by no means exhaustive and its order is not an indication of a preferred order of use. It will be understood that a successful purification of HAV capsids may include any, some, all, or none of steps (a)-(m) as these steps are optional and do not effect the outcome of the process of the present invention deliniated above.

Additional processing steps of conventional and well known character are or may be needed to prepare purified HAV capsids for vaccine use. For example, treatment with formalin, sterile filtration and adsorption to carriers or adjuvants are the typical basic steps for preparing a formalin-inactivated vaccine. See, for example, Provost, P.J. et al. Proc. Soc. Exp. Biol. Med. 160, 213 (1979); Provost, P.J. et al. J. Med Virol. 19,23 (1986). HAV can be inactivated by heat, pH changes, treatment with organic solvents, ultraviolet irradiation, or exposure to formalin. It will be understood that the scope of the present invention encompasses, in addition to the P18 variant of strain CR326F of HAV, any other HAV variant or strain, whether attenuated or virulent. Attenuated variants or strains may be isolated by serial passage in cells, animals, or by other methods. See, for example, Provost, P.J. et al. Proc. Soc. Exp. Biol. Med. 170,8 (1982); and Provost, P.J. et al. J. Med. Virol. 20, 165 (1986), for details on attenuation. The purification methods of the present invention are readily and easily adaptable to attenuated or virulent HAV strains.

The example that follows illustrates the practice of the invention, but it is not intended to limit the scope and content of the invention.

EXAMPLE

Procedure for the Purification of Lepatitis A Virus (HAV) Capsids from MRC-5 Cells

A. Cell Disruption

MRC-5 cells infected with an attenuated HAV (variant P18 of strain CR326F) were harvested by scraping the cells from the culture substratum, collecting the cells in polypropylene tubes, and freezing at -70°C, at four roller bottle equivalents of cells per tube. Two tubes were thawed by the addition of 3 mL of lysis buffer per tube (10 mM TRIS-HCl pH 7.5, 10 mM NaCl, 1.5 mM $MgCl_2$) and by vigorous vortexing (10-15 seconds at setting 10 in a Vortex-Genie) and were then held on wet ice for 15 minutes. The lysates were twice frozen in an ethanol-dry ice bath and thawed in a 37°C water bath, and each tube was sonicated three times for 30 seconds at the maximum wattage output of a cup horn sonicator with a circulating ice water bath (Branson sonifier Cell Disrupter 185 and Ultrasonics cup horn). The sonicates were pooled and assayed for protein using a Bradford protein assay (BioRad) with BSA (Bovine Serum Albumin) as the protein concentration standard, and then adjusted to 3 mg protein per mL using TNE buffer (10 mM TRIS-HCl pH 7.5, 150 mM NaCl, 1 mM EDTA). The sonicate was organically extracted by the addition of an equal volume of methylene chloride:isoamyl alcohol (24:1,v/v) with vortexing for 1 minute and was centrifuged at 3,000 rpm for 10 minutes at 20°C in an IEC centrifuge (3,000 x g) to achieve organic/aqueous phase separation. The aqueous phase was reserved, the organic phase discarded and the interface was reextracted with a volume of TNE buffer equal to one third of the original sample volume, by vortexing and centrifugation as above. The two aqueous phases were pooled and the volume measured, yielding HAV capsids in organically extracted cell lysates.

B. Polyethylene glycol (PEG) precipitation

HAV capsids in the organically extracted cell lysates were concentrated by polyethylene glycol precipitation (PEG). Lysates were adjusted to 500 mM NaCl and then made 4% (w/v) of PEG 8000 by the addition, with vigorous vortexing, of 40% (w/v) PEG 8000 (prepared in 10 mM TRIS-HCl pH 7.5, 500 mM NaCl, 1 mM EDTA). The 4% PEG lysates were held at 4°C for 1 hour and then centrifuged at 1,500 x g for 10 minutes at 4°C in a Beckman HB-4 rotor. The supernatant was removed and the PEG pellet resuspended by vigorous vortexing in 10 mL of 10 mM TRIS-HCl pH 7.5, 50 mM NaCl, 10 mM EDTA, and by sonication 3 times for 30 seconds each time at maximum wattage output in a cup horn sonicator with a circulating ice water bath as described above. The sonicated, resuspended PEG pellet was organically extracted by the addition of an equal volume of chloroform:isoamyl alcohol (24:1,v/v) with vigorous vortexing, and then clarified by centrifugation at 4,000 x g for 10 minutes at 20°C. The aqueous phase was reserved, the interface and organic phase were reextracted with a volume of TNE equal to one third of the sonicated, resuspended PEG pellet volume and both aqueous phases were combined, yielding resuspended PEG pellet.

C. Ion exchange and Gel filtration chromatography

The resuspended PEG pellet was adjusted to 0.35 M NaCl and chromatographed through two 2 mL DEAE-Sepharose CL-6B (Pharmacia) columns linked in series, at a pump flow rate of 3 mL/minute. The column was prepared in Kontes Flex column (1.0 x 5.0 cm) and equilibrated in 10 mM TRIS-HCl pH 7.5, 0.35 M NaCl, 1 mM EDTA. The first 15 mL of eluate, which contain the HAV capsids, were collected. The eluate was diluted to a NaCl concentration of 0.15 M using 10 mM TRIS, 1 mM EDTA, pH 7.5, and then chromatographed through a 2 mL DEAE-Sepharose CL-6B (Pharmacia) column at a pump flow rate of 3 mL/minute. The column was prepared in a Kontes Flex column (1.0 cm x 5.0 cm) and equilibrated in 10 mM TRIS, 0.15 M NaCl, 0.1 mM EDTA, pH 7.5. The column was washed with several column volumes of 10 mM TRIS, 0.15 M NaCl, 0.1 mM EDTA, pH 7.5. HAV capsids were eluted using 10 mM TRIS, 0.35 M NaCl, 1 mM EDTA, pH 7.5. The first 15 mL of eluate, which contains the HAV capsids, were collected.

The eluate was then chromatographed through a Sepharose CL-4B (Pharmacia) column (2.6 x 95 cm), equilibrated in TNE buffer, and operated at pump flow rate of 40 mL/hour. Fractions of five mL were collected, yielding substantially purified, attenuated HAV capsids. Fractions containing HAV capsids were identified by radio immuno assay (RIA) using an antibody specific for HAV, combined, sterilized by filtration through a Millex GV-0.22 µ filter (Millipore) and stored at -70°C. The concentration of HAV in the pool was determined by quantitative RIA and the sample concentrated (if necessary) by lyophilization.

With a sample of 5 µg of purified HAV capsids, SDS-polyacrylamide gel electrophoresis failed to detect any impurities after silver staining and Western blotting.

While the foregoing specification teaches the principles of the present invention, with examples provided for the purpose of illustration, it will be understood that the practice of the invention encompasses all of the usual variations, adaptations, modifications, deletions or additions of procedures and protocols described herein, as come within the scope of the following claims.

**Claims**

1. A method of substantially purifying HAV capsids comprising the steps of
   (a) subjecting the aqueous phase of a lysate of HAV infected cells to ion exchange chromatography;
   (b) subjecting the effluent of (a) to ion exchange chromatography; and
   (c) gel filtering the eluate of (b) containing HAV capsids, yielding substantially purified HAV capsids.

2. The product of the process of Claim 1.

3. Hepatitis A virus capsids in substantially pure form free of contaminating 30nm particles when observed by electron microscopy.

4. The method of Claim 1 wherein the ion exchange chromatography of steps (a) and (b) is anion exchange chromatography.

5. The method of Claim 4 wherein the anion exchange chromatography of step (a) is done at a NaCl concentration of between approximately 0.3 M and 0.4 M.

6. The method of Claim 5 wherein the anion exchange chromatography of step (a) is done at a NaCl concentration of about 0.35 M.

7. The method of Claim 4 wherein the anion exchange chromatography of step (b) is done at a NaCl concentration between approximately 0.1 M and 0.2 M for HAV capsid adsorption, and a NaCl concentration between 0.3 M and 0.4 M for HAV capsid elution.

8. The method of Claim 7 wherein the NaCl concentration is about 0.15 M for HAV capsid adsorption and the NaCl concentration for HAV capsid elution is about 0.38 M.

9. A method of substantially purifying attenuated HAV capsids for vaccination purposes, comprising the steps of:
   (a) subjecting the aqueous phase of a lysate of HAV infected cells to anion exchange chromatography at 0.35 M NaCl;
   (b) subjecting the effluent of (a) to anion exchange chromatography with adsorption at 0.15 M NaCl, and elution at 0.38 M NaCl; and
   (c) gel filtering the eluate of (b) containing HAV capsids, yielding substantially purified attenuated HAV capsids for vaccination purposes.

10. The product of the process of Claim 9.

# FIG. 1

# FIG. 2